# EUROPEAN PATENT APPLICATION

(11) **EP 2 298 881 A1**
(43) Date of publication of application: **23.03.2011**
(21) Application number: 09750632.3
(22) Date of filing: 21.05.2009
(51) Int. Cl.: C12N 15/09, C12Q 1/68

(54) **NUCLEIC ACID MOLECULE HAVING THE CAPACITY TO BOND WITH A 2,4,6-TRINITROPHENYL STRUCTURE, METHOD FOR DETECTING COMPOUNDS INCLUDING A 2,4,6-TRINITROPHENYL STRUCTURE USING SAID NUCLEIC ACID MOLECULE, AND USE OF SAID NUCLEIC ACID MOLECULE**

(30) Priority: 21.05.2008 JP 2008133263
(71) Applicant: NEC Soft, Ltd., Tokyo 136-0082 (JP)
(72) Inventor: YOSHIDA, Yoshihito, Tokyo 136-0082 (JP); HORII, Katsunori, Tokyo 136-0082 (JP); AKITOMI, Jou, Tokyo 136-0082 (JP); FURUICHI, Makio, Tokyo 136-0082 (JP); WAGA, Iwao, Tokyo 136-0082 (JP)
(74) Representative: Vossius & Partner
(86) International application number: PCT/JP2009/059367
(87) International publication number: WO 2009/142270

(57) **Abstract**

The present invention relates to a nucleic acid molecule capable of binding to a 2,4,6-trinitrophenyl skeleton, a method for detecting a compound having the 2,4,6-trinitrophenyl skeleton using the nucleic acid molecule, use of the nucleic acid molecule for detecting a compound having the 2,4,6-trinitrophenyl skeleton, and a method for detecting a compound having the 2,4,6-trinitrophenyl skeleton.

## Description

### Technical Field

The present invention relates to a nucleic acid molecule capable of binding to a 2,4,6-trinitrophenyl skeleton, a method for detecting a compound having the 2,4,6-trinitrophenyl skeleton using this nucleic acid molecule, and use of this nucleic acid molecule.

### Background Art

Trinitrotoluene (2,4,6-trinitrotoluene) is a compound having a structure represented by the following structural formula (I). It generally is used as so-called TNT gunpowder.

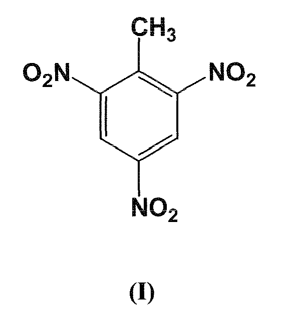

A method for detecting TNT is a life-related important matter and is a technology attracting attention. For example, Patent Document 1 discloses a method for detecting a nitro compound using a complex having a predetermined siloxane repeating unit. Patent Document 2 discloses a system for detecting a low molecular weight compound such as TNT extracted via a charged surface of a collector using detection means such as mass spectrometry. Patent Document 3 discloses a method for detecting TNT with the use of a member obtained by binding TNT that reversibly binds to an antibody specific to TNT to a surface of a metal coating a solid carrier such as a piezoelectric crystal electrode via a linker molecule of aliphatic hydrocarbon.

However, the methods disclosed in these documents have both good and bad points in terms of requirements demanded currently. Specifically, in the method disclosed in Patent Document 1, a monomer having a predetermined siloxane repeating unit necessary for the detection needs to be polymerized, and further, the obtained polymer needs to be processed into a thin film. Thus, construction of a detection system is very difficult. In the method disclosed in Patent Document 2, an object to be detected needs to be ionized, which places the restriction on the form of the object to be detected. In the method disclosed in Patent Document 3, it is necessary to prepare an antibody that specifically binds to an object to be detected. Thus, there is severe restriction on the productivity of the antibody. Also, the method has an ethical problem because animals need to be used for preparation of the antibody.

The form in which TNT as an object to be detected is present is affected by the physical properties and the like of a sample containing the object. Furthermore, a system that can detect even a trace amount of TNT is preferable. However, none of the methods disclosed in these documents satisfies these requirements.

Moreover, in any of the methods disclosed in these documents, TNT as an object to be detected needs to be treated directly in a detection system, which is dangerous to those who conduct the method.

Therefore, it cannot be said that these methods satisfy the requirements demanded currently for TNT detection.

### Prior Art Documents

### Patent Documents

Patent Document 1: JP 2007-513347 A
Patent Document 2: JP 2007-515619 A
Patent Document 3: JP 2005-530175 A

### Non-Patent Documents

Non-Patent Document 1: M. Zuker, Science, 1989, vol. 244, pp.48-52

### Disclosure of the Invention

### Problem to be Solved by the Invention

The present invention has been made in light of the above-described conventional problems. It is an object of the present invention to provide: a nucleic acid molecule that places no restriction on the form of a sample containing an object to be detected, can be produced with high reproducibility, and has a high capability to bind to compounds having a 2,4,6-trinitrophenyl skeleton, such as TNT; a method for detecting a compound having the 2,4,6-trinitrophenyl skeleton using this nucleic acid molecule; and use of this nucleic acid molecule in the detection.

### Means for Solving Problem

A first aspect of the present invention relates to a nucleic acid molecule capable of binding to a 2,4,6-trinitrophenyl skeleton.

A second aspect of the present invention relates to a method for detecting a compound having a 2,4,6-trinitrophenyl skeleton, wherein the nucleic acid molecule capable of binding to the 2,4,6-trinitrophenyl skeleton is used.

A third aspect of the present invention relates to use of the nucleic acid molecule capable of binding to a 2,4,6-trinitrophenyl skeleton to detect a compound having the 2,4,6-trinitrophenyl skeleton.

### Effects of the Invention

According to the present invention, it becomes possible to provide a nucleic acid molecule that can detect compounds having the 2,4,6-trinitrophenyl skeleton, including TNT as a raw material of gunpowder, with high sensitivity conveniently and safely.
Furthermore, according to the detection method using the nucleic acid molecule of the present invention and the use of the nucleic acid molecule, compounds having the 2,4,6-trinitrophenyl skeleton, including TNT, can be detected with high sensitivity conveniently and safely.

### Brief Description of Drawings

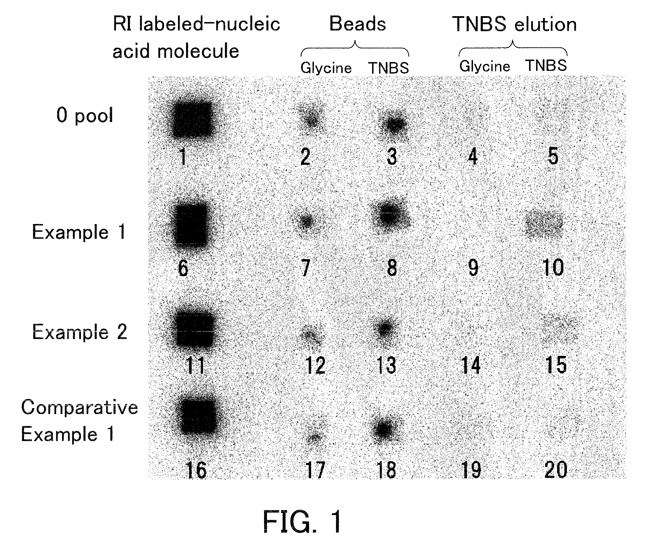

[FIG. 1] FIG. 1 shows a dot blot image.

### Mode for Carrying Out the Invention

### (Nucleic acid molecule according to the present invention)

The nucleic acid molecule according to the present invention is characterized in that it is capable of binding to the 2,4,6-trinitrophenyl skeleton.

In the present invention, the nucleic acid molecule is not particularly limited as long as it is a nucleotide containing various nucleic acids such as adenine (A), guanine (G), cytosine(C), thymine (T), and uracil (U), and there is no limitation on: the number of strands, i.e., whether the nucleic acid molecule is ssDNA, ssRNA, dsDNA, dsRNA, or the like; whether or not the nucleic acid is modified; and the like. Furthermore, the nucleic acid molecule also encompasses substitution products thereof resultant from suitable substitution with halogens such as fluorine, chlorine, bromine, and iodine and alkyl groups such as methyl, ethyl, and propyl, as long as the substitution does not affect the degree of binding with the 2,4,6-trinitrophenyl skeleton.

The nucleic acid molecule according to the present invention is a nucleic acid molecule capable of binding to the 2,4,6-trinitrophenyl skeleton, and it preferably includes a base sequence substantially having a homology to the base sequence of SEQ ID NO: 6 or 7. In the present invention, "a base sequence substantially having a homology" means: (1) a base sequence obtained by deletion, substitution, and/or addition of one or more bases in a base sequence to be compared therewith (e.g., the base sequence of SEQ ID NO: 6 or 7); or (2) a base sequence having a homology of at least 70% to the base sequence to be compared therewith. Furthermore, it is more preferable that a part or whole of the nucleic acid molecule of the present invention is composed of a base sequence having a homology of at least 80%, more preferably at least 90%, still more preferably at least 95%, and most preferably at least 99% to the base sequence of SEQ ID NO: 6 or 7.

Furthermore, the nucleic acid molecule according to the present invention is a nucleic acid capable of binding to the 2,4,6-trinitrophenyl skeleton, and it preferably has substantially the same putative structure and/or structural motif. In the present invention, "having substantially the same putative structure and/or structural motif" means that, through observation using a program for predicting the secondary structure of a nucleic acid sequence and the motif of this structure, a certain degree of identity is found in a sequence group consisting of a plurality of sequences. When such a certain degree of identity is found, it is preferable that the homology among the sequences compared with one another is at least 70%. By substantially having the identity, the nucleic acid molecule according to the present invention can exhibit an improved binding property to the 2,4,6-trinitrophenyl skeleton. Examples of such a program include the Zukerfold program described in Non-Patent Document 1.

An example where the nucleic acid molecule of the present invention has substantially the same putative structure and/or structural motif is as follows. SEQ ID NO: 6 in the present invention includes a structural motif "GCGAGAA". As described in examples of the present invention, when an aptamer of SEQ ID NO: 6 was obtained by the SELEX method to be described later, 45 sequences in the RNA pool obtained finally were analyzed. As a result, it was found that this structural motif was contained in nine sequences out of the 45 sequences. Furthermore, out of these nine sequences, seven sequences including SEQ ID NO: 6 were subjected to the analysis of the secondary structure of the nucleic acid molecule. As a result, it was predicted that each of these sequences had a stem-loop structure, and in the secondary structure of each of these sequences, *the "GCGA GAA" motif was present so as to overlap with the stem portion and the loop portion with the stem portion being flanked.* When the nucleic acid molecule of the present invention has substantially the same putative structure and/or structural motif as described above, it becomes possible to ensure the capability of the nucleic acid molecule to bind to the 2,4,6-trinitrophenyl skeleton. Therefore, for example, it is preferable that whole or a part of the nucleic acid molecule of the present invention includes a base sequence having a homology of at least 80%, preferably at least 90%, and more preferably at least 95% to the base sequence of SEQ ID NO: 6 and has a structural motif "GCGAGAA". Furthermore, in this case, it is more preferable that, in the predicted stem-loop structure, *the structural motif "GCGAGAA" overlaps with the stem portion and the loop portion with the stem portion being flanked.*

In the present invention, the "2,4,6-trinitrophenyl skeleton" refers to a structure represented by the following structural formula (II).

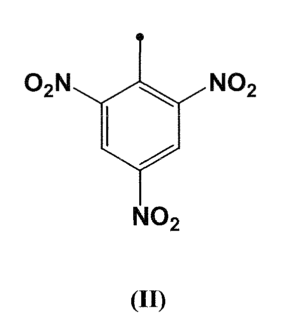

The nucleic acid molecule of the present invention can be produced by a method in which, using nucleic acid molecules such as so-called RNA pools and a suitable base material having the 2,4,6-trinitrophenyl skeleton as a target substance, a nucleic acid molecule-target substance complex formed through specific binding of a nucleic acid molecule with the target substance is obtained, and only a nucleic acid molecule involved in the formation of this complex is selected from this complex. Examples of such a method include a method called the SELEX (Systematic Evolution of Ligands by Exponential Enrichment) method and a method in which, after a nucleic acid molecule-target substance complex is obtained using a carrier such as an agarose gel or a polyacrylamide gel, only a nucleic acid molecule involved in the formation of this complex is collected.

### (Method for producing nucleic acid molecule of the present invention based on SELEX method)

The nucleic acid molecule of the present invention can be produced, according to the SELEX method or a method analogous thereto, by causing a reaction of RNA pools and a suitable base material having a target substance, collecting an RNA pool-target substance complex obtained through the reaction, and then, from this complex, collecting only an RNA pool involved in the formation of this complex.

The term "RNA pool" means a gene mixture and collectively refers to a gene sequence having a region where bases selected from the group consisting of A, G, C, and U and substitution products of these bases are linked so that the total number thereof is about 20 to 120 (this region hereinafter is referred to as "random region"). Therefore, the RNA pool contains 4²⁰ to 4¹²⁰ (10¹² to 10⁷²) kinds of genes, preferably 4³⁰ to 4⁶⁰ (10¹⁸ to 10³⁶) kinds of genes. Examples of the substitution products of the bases include those obtained by suitably substituting the bases with halogens such as fluorine, chlorine, bromine, and iodine and alkyl groups such as methyl, ethyl, and propyl.

As long as the RNA pool has a random region, other structures thereof are not limited. However, in the case where the nucleic acid molecule of the present invention is produced based on the SELEX method, it is preferable that the RNA pool has a primer region to be used in PCR or the like to be described below and a DNA-dependent RNA polymerase recognition region in the 5'-end portion and/or 3'-end portion of the random region. For example, the structure of the RNA pool may be such that, from the 5'-end side thereof, a DNA-dependent RNA polymerase recognition region such as a T7 promoter (hereinafter, this region is referred to as "RNA polymerase recognition region") and a primer region for a DNA-dependent DNA polymerase (hereinafter, this region is referred to as "5'-end side primer region") are linked, a random region is linked to the 3'-end of this 5'-end side primer region, and further a primer region for a DNA-dependent DNA polymerase (hereinafter, this region is referred to as "3'-end side primer region") is linked to the 3'-end side of this random region. Furthermore, the RNA pool may have, in addition to these regions, a known region that assists the binding to a target substance. Still further, the sequence of a part of the random region may be common to respective RNA pools.

The random region may be prepared by conducting gene amplification based on a PCR method with an initial pool obtained by substituting U in the random region of the RNA pool with T as a template and then causing the resultant gene product to react with a DNA-dependent RNA polymerase such as T7 polymerase. Alternatively, the random region may be prepared based on the PCR method by synthesizing a gene complementary to the initial pool and annealing a primer composed of a sequence complementary to the RNA polymerase recognition region and the 5'-end side primer region to a gene complementary to this primer in the initial pool.

A base material having the 2,4,6-trinitrophenyl skeleton as a target substance may be selected within a range where no problem is caused in obtaining the nucleic acid molecule capable of binding to the 2,4,6-trinitrophenyl skeleton in the following manner. Examples of the base material include beads and fibers. Examples of a material for forming the base material include cellulose, sepharose, and agarose. Furthermore, by immobilizing a target substance on a protein, selection using a filter such as nitro cellulose becomes possible.

A material for providing the 2,4,6-trinitrophenyl skeleton in the preparation of a base material having the 2,4,6-trinitrophenyl skeleton as a target substance is not particularly limited as long as it is any of various kinds of materials having reactivity with the base material. Examples of the material include compounds having the 2,4,6-trinitrophenyl skeleton, such as TNBS (2,4,6-trinitrobenzenesulfonic acid).

The binding between the 2,4,6-trinitrophenyl skeleton and the base material preferably is achieved in the form of covalent bond from the viewpoint of stability. Furthermore, for the binding of the 2,4,6-trinitrophenyl skeleton and the base material, a suitable linker molecule may be used. Examples of such a linker molecule include compounds containing both an amino group and a carboxyl group, such as glycine. When the 2,4,6-trinitrophenyl skeleton and the base material are bound to each other using a linker molecule, the resultant target substance is, for example, a 2,4,6-trinitrophenyl skeleton-linker molecule-base material. When glycine is used as a linker molecule, the carboxyl terminus of the glycine may be used for the binding with the base material and the amino terminus of the glycine may be used for the binding with the 2,4,6-trinitrophenyl skeleton.

Next, the thus-synthesized RNA pool and a suitable base material having the 2,4,6-trinitrophenyl skeleton as a target substance are bound to each other via intermolecular force such as hydrogen bond. Examples of this binding method include a method in which the RNA pool and the target substance are incubated for a certain period of time in a buffer solution in which a function such as the binding with the target substance is maintained. In this manner, an RNA pool-target substance complex is formed in the buffer solution.

Next, the thus-formed RNA pool-target substance complex is collected. The buffer solution contains, in addition to this complex, RNA pools and target substances that have not been involved in the formation of the complex. The method for collecting this complex can be carried out by removing random regions that have not been involved in the formation of the complex in the buffer solution with the aim of collecting a nucleic acid molecule having a binding property to the target substance. Examples of this method include a method utilizing the binding property between the RNA pool and the target substance in the RNA pool-target substance complex, a method utilizing the difference in molecular weight between the complex and the RNA pool, and a method utilizing the difference in adsorbability between the target substance and the RNA pool.

Examples of the method utilizing the binding property between the RNA pool and the target substance in the RNA pool-target substance complex include methods utilizing various kinds of bond formed between the RNA pool and the target substance, such as hydrogen bond. For example, when sepharose beads to which the 2,4,6-trinitrophenyl skeleton represented by the above formula (II) as a target substance is bound through covalent bond are used, it is possible to use a method in which a solvent containing RNA pools is applied to the beads, and then, from an RNA pool-target substance complex obtained by the binding of an RNA pool to the beads via the 2,4,6-trinitrophenyl skeleton, the RNA pool is collected under the conditions where the binding between the RNA pool and the target substance is cleaved. The conditions where the binding between the RNA pool and the target substance in the RNA pool-target substance complex is cleaved may be selected as appropriate considering the form of the binding between the RNA pool and the target substance. For example, a solution having a chaotropic effect, such as a solution of urea or guanidine hydrochloride, which cleaves hydrogen bond, may be used, or EDTA (ethylenediamine tetraacetic acid salt), EGTA (glycoletherdiamine tetraacetic acid salt), or the like, which chelates a divalent metal, such as Mg²⁺, necessary for the binding between the RNA pool and the target substance may be used. Furthermore, a compound that has the 2,4,6-trinitrophenyl skeleton and competes with the binding between the RNA pool and the target substance in the RNA pool-target substance complex may be used. Examples of such a compound include trinitro compounds such as TNT, TNBS (2,4,6-trinitrobenzenesulfonic acid), and picric acid (2,4,6-trinitrophenol (TNF)). Examples of such a compound further include dinitro compounds such as dinitrotoluene (2,4-DNT or 2,6-DNT). Note here that these conditions may be used alone or in appropriate combination.

Furthermore, examples of the method utilizing the difference in molecular weight between the RNA pool-target substance complex and the RNA pool include a method in which, utilizing a carrier, such as agarose gel, having pores that allow the RNA pool to pass therethrough but does not allow the RNA pool-target substance complex to pass therethrough, the RNA pool is electrically separated from the RNA pool-target substance complex, thus collecting the RNA pool involved in the formation of the complex from this complex.

Still further, as the method utilizing the difference in adsorbability between the target substance and the RNA pool, the selection utilizing a nitrocellulose membrane becomes possible by immobilizing a protein labeled with TNBS as the target substance. A buffer solution containing the above-described RNA pool-target substance complex is filtered through the membrane that can adsorb the target substance, thereby causing the RNA pool-target substance complex to be adsorbed on this membrane. Thereafter, from the RNA pool-target substance complex remaining on this membrane, the RNA pool involved in the formation of the complex is collected, for example, after the RNA pool and the target substance in this complex are unbound.

Next, gene amplification is carried out using the thus-obtained RNA pool that has been involved in the formation of the complex and collected from the RNA pool-target substance complex. Examples of the method for carrying out this gene amplification include a method utilizing a 5'-end side primer region, a 3'-end side primer region, and a RNA polymerase recognition region contained in the RNA pool. For example, gene amplification of the RNA pool may be carried out in the following manner. Using a gene fragment complementary to the 3'-end side primer region of the RNA pool involved in the formation of the complex as a primer, cDNA is prepared by a reverse transcription reaction of an RNA-dependent DNA polymerase such as avian myeloblastosis virus-derived reverse transcriptase (AMV Reverse Transcriptase). Thereafter, utilizing a 5'-end side primer region and a 3'-end side primer region contained in this cDNA, a PCR reaction using a DNA-dependent DNA polymerase is carried out. Then, utilizing an RNA polymerase recognition region contained in the thus-obtained gene product, an in vitro transcription reaction is carried out using a DNA-dependent RNA polymerase.

Using the RNA pool that has been involved in the formation of the complex and subjected to the above-described gene amplification and the target substance, respective processes subsequent to the above-described process for forming the RNA pool-target substance complex are repeated. This allows a nucleic acid molecule that specifically binds to a suitable base material having the 2,4,6-trinitrophenyl skeleton as a target substance, i.e., a nucleic acid molecule capable of binding to the 2,4,6-trinitrophenyl skeleton, to be obtained finally.

In the example given above, the nucleic acid molecule of the present invention is produced by the method based on the SELEX method. Also, as another example, the nucleic acid molecule of the present invention may be produced by, for example, chemically synthesizing a nucleic acid molecule capable of binding to the 2,4,6-trinitrophenyl skeleton through modification such as deletion, substitution, and/or addition of a base(s) in the base sequence represented by SEQ ID NO: 6 or 7, whose capability to bind to the 2,4,6-trinitrophenyl skeleton has been demonstrated in the following examples. The capability of the thus-obtained nucleic acid molecule to bind to the 2,4,6-trinitrophenyl skeleton can be evaluated by the method described in the following examples and other known methods.

### (Method for detecting compound having 2,4,6-trinitrophenyl skeleton according to the present invention and use of nucleic acid molecule of the present invention)

A method for detecting a compound having the 2,4,6-trinitrophenyl skeleton and use of the nucleic acid molecule according to the present invention are characterized in that a nucleic acid molecule capable of binding to the 2,4,6-trinitrophenyl skeleton is used to detect a compound having the 2,4,6-trinitrophenyl skeleton (2,4,6-trinitrophenyl skeleton-containing compound). That is, a nucleic acid molecule to be used in the detection method and the use of a nucleic acid molecule according to the present invention may be the above-described nucleic acid molecule of the present invention.

When the nucleic acid molecule of the present invention binds to a compound having the 2,4,6-trinitrophenyl skeleton, examples of a method for detecting the binding include methods utilizing color development, fluorescence, chemiluminescence, and the like and methods including the use of an electric sensor, and commonly-used known techniques can be applied as appropriate. For example, by using the nucleic acid molecule of the present invention in the state where it is immobilized on a suitable solid phase, the change in RNA structure caused by the binding of TNT thereto can be detected by the change in electric potential or fluorescence. More specifically, one example of the detection according to the present invention may be such that RNA labeled with an electron-donating substance such as methylene blue is immobilized on a gold membrane, and the detection is achieved by measuring the change in electric potential caused when the structure of the RNA is changed by the binding of TNT thereto. Alternatively, the detection may be such that RNA is modified using both a fluorescent substance and a substance for quenching fluorescence caused by the fluorescent substance, and the change in structure of this modified RNA caused by the binding of TNT thereto is measured by measuring the change in fluorescence.

In the detection method or the use of a nucleic acid molecule according to the present invention, a compound having the 2,4,6-trinitrophenyl skeleton as an object to be detected is not particularly limited, and examples thereof include TNT, 2,4,6-trinitrobenzenesulfonic acid, 2,4,6-trinitroanisole, triaminotrinitrobenzene, and picric acid.

The nucleic acid molecule of the present invention can be used for the detection of TNT having the 2,4,6-trinitrophenyl skeleton and the like in various applications. Examples of such applications include test reagents, sensors, and capturing a target molecule with the nucleic acid molecule bound to a filtration filter or the like.

### Examples

### (Production Example 1)

### [Preparation of RNA pool]

An initial pool represented by SEQ ID NO: 4 was synthesized using a DNA synthesizer (334 DNA synthesizer (Applied Biosystems)). Using this initial pool (500 nM), a primer 1 (SEQ ID NO: 2), a primer 2 (SEQ ID NO: 3), and 2.5 U of DNA polymerase (trade name: Ex-Taq, Takara Bio Inc.), cDNA composed of the initial pool and a gene strand complementary to the initial pool was obtained. Next, a transcription reaction was carried out using the thus-obtained cDNA and T7 RNA polymerase (trade name: Ampliscribe (EPICENTRE)). Thus, an RNA pool (SEQ ID NO: 1) was obtained.

### [Preparation of TNBS-immobilized beads]

0.5 ml of 0.2M glycine (Wako) dissolved in 0.1M MES (2-(N-morpholino)ethanesulfonic acid) (pH4.7) and 0.5 ml of 0.2M 2,4,6-trinitrobenzenesulfonic acid sodium salt (hereinafter abbreviated as "TNBS") (Wako) dissolved in 100% DMSO were caused to react at 4°C for 24 hours. Thus, a reaction solution was obtained. Next, the reaction solution was mixed with 10 ml of EAH Sepharose 4B (GE). The resultant mixture was allowed to react at 4°C for 24 hours. Thus, TNBS-immobilized beads were obtained.

### [Binding of RNA pool and TNBS-immobilized beads]

The TNBS-immobilized beads obtained in the above-described manner were suspended in a binding buffer (50 mM HEPES (pH 7.4), 500 mM NaCl, 5 mM MgCl₂). This was packed into Ultra-free MC (MILLIPORE) and equilibrated with a suitable amount of the binding buffer. The RNA pool prepared in the above-described manner was dissolved in the binding buffer, and the resultant mixture was applied to the equilibrated column, thereby binding the RNA pool to the TNBS-immobilized beads.

After binding the RNA pool to the TNBS-immobilized beads, a binding buffer containing 7 M urea (hereinafter referred to as "elution buffer 1") was caused to flow through the column to obtain an eluate.

Thereafter, using this eluate (corresponding to 20 µM of RNA), a primer 3 (SEQ ID NO: 5), and AMV-derived reverse transcriptase Transcriptor (Roche), a reverse transcription reaction was carried out at 55°C for 30 minutes.

Using the whole of this reaction product, 2.5 U of DNA polymerase (trade name: Ex-Taq, Takara Bio Inc.), 30 nM of the primer 1 (SEQ ID NO: 2), and 30 nM of the primer 2 (SEQ ID NO: 3), a PCR reaction with 12 cycles was conducted with a treatment at 90°C for 50 seconds, 53°C for 70 seconds, and 74°C for 50 seconds in this order as one cycle. The resultant solution was subjected to ethanol precipitation, thus obtaining a double-stranded DNA product.

This double-stranded DNA product was dissolved in 8 µl of RNase-free water. Using 4 µl of the resultant mixture and 16 µl of a T7 RNA polymerase solution (trade name: Ampliscribe (EPICENTRE)), in vitro transcription was carried out. Thus, an in vitro transcript was obtained. The steps performed up to here are defined as one cycle.

Then, the above-described series of operations were repeated in accordance with Table 1 showing the elution condition used in each cycle, and a nucleic acid molecule represented by SEQ ID NO: 6 was obtained finally. In Table 1, "elution buffer 1" is the above-described elution buffer 1, "elution buffer 2" is a binding buffer containing 10 mM EDTA, and "-" indicates that elution was not conducted in the corresponding cycle and the elution in the cycle immediately before that cycle was the final elution.

In the present Production Example 1, 45 sequences including SEQ ID NO: 6 in the DNA pool obtained after being subjected to the ten cycles were analysed. As a result, it was found that a structural motif "GCGAGAA" was contained in nine sequences out of these 45 sequences. Furthermore, out of these nine sequences, seven sequences including SEQ ID NO: 6 were subjected to the analysis of the secondary structure of the nucleic acid molecule. As a result, it was predicted that each of these sequences had a stem-loop structure, and in the secondary structure of each of these sequences, *the structural motif "GCGAGAA" was present so as to overlap with the stem portion and the loop portion with the stem portion being flanked.*

### (Production Example 2)

The same procedure as in Production Example 1 was performed except that the elution in Production Example 1 was conducted under the elution condition shown in Table 1. Thus, a nucleic acid molecule of SEQ ID NO: 7 was obtained.

**[Table 1]**

| Cycle | Production Example 1 | Production Example 2 |
|---|---|---|
| 1 | elution buffer 1 | elution buffer 2 |
| 2 | elution buffer 1 | elution buffer 2 |
| 3 | elution buffer 1 | elution buffer 2 |
| 4 | elution buffer 1 | elution buffer 2 |
| 5 | elution buffer 1 | elution buffer 1 |
| 6 | elution buffer 1 | elution buffer 1 |
| 7 | elution buffer 1 | elution buffer 1 |
| 8 | elution buffer 1 | elution buffer 1 |
| 9 | elution buffer 1 | elution buffer 1 |
| 10 | elution buffer 1 | - |

### (Example 1)

The TNBS-immobilized beads obtained in Production Example 1 were packed into a column in the same manner as in Production Example 1. On the other hand, using the above-described double-stranded DNA product, a transcription reaction was carried out in the presence of α-³²P-ATP (Amersham Biosciences) based on the above-described in vitro transcription method, thereby radiolabeling the sequence represented by SEQ ID NO: 6. Thus, an RI labeled-in vitro transcript was obtained. This RI labeled-nucleic acid molecule was added to the column packed with the beads, whereby the RI labeled-nucleic acid molecule was bound to the beads. The beads were washed using a sufficient amount of binding buffer, thus obtaining a complex of the RI labeled-nucleic acid molecule and the TNBS-immobilized beads. A binding buffer containing 10 mM TNBS and 5 mM MgCl₂ was added to this complex, thereby eluting the complex from the column. It is speculated that this eluate contained the RI labeled nucleic acid molecule that specifically binds to TNBS.

Moreover, as a control group, beads were prepared by conducting the procedure described in the above [Preparation of TNBS-immobilized beads] section without using TNBS (hereinafter the thus-prepared beads are referred to as "glycine beads"), and a complex and concentrated eluate were obtained in the same manner as in the above using these glycine beads.

These complex and eluate were bound to Whatman 3M filter paper based on the dot blot method, and RI detected on the filter paper was observed. The result thereof is shown in FIG. 1. In FIG. 1, "0 pool" indicates a product obtained in the same manner as in the above using the RNA pool represented by SEQ ID NO: 1.

Also, Table 2 shows the proportion of a value obtained by converting each dot density to a numerical value by a densitometer, assuming that a value obtained by converting the dot density corresponding to the RI labeled nucleic acid molecule used for the formation of the complex to a numerical value by a densitometer was 100%. In Table 2, "dot number" indicates the number shown below each of the dots in FIG. 1.

### (Example 2)

A complex and eluate were obtained in the same manner as in Example 1, except that the sequence of SEQ ID NO: 7 was used instead of the sequence of SEQ ID NO: 6. The result is shown in FIG. 1 and Table 2.

**[Table 2]**

| | Dot number | Proportion (%) |
|---|---|---|
| Example 1 | 6 | 100 |
| | 7 | 3.5 |
| | 8 | 22.0 |
| | 9 | 1.0 |
| | 10 | 3.3 |
| Example 2 | 11 | 100 |
| | 12 | 2.5 |
| | 13 | 5.8 |
| | 14 | 1.1 |
| | 15 | 2.1 |
| Comparative Example 1 | 16 | 100 |
| | 17 | 2.3 |
| | 18 | 13.3 |
| | 19 | 1.2 |
| | 20 | 1.3 |

### (Comparative Example 1)

An RI-labeled product was obtained in the same manner as in Example 1, except that the double-stranded DNA product obtained at the 6th cycle in Production Example 1 was used instead of the sequence of SEQ ID NO: 6. Using the thus-obtained RI-labeled product, a complex and eluate were obtained in the same manner as in Example 1. The result is shown in FIG. 1 and Table 2.

### Industrial Applicability

According to the present invention, gunpowders and explosives, such as trinitrotoluene, can be detected conveniently and safely. Hence, the present invention has high industrial applicability in the fields of chemical industry, security measures, and the like.

[Sequence Listing]

## Claims

**1.** A nucleic acid molecule capable of binding to a 2,4,6-trinitrophenyl skeleton.

**2.** The nucleic acid molecule according to claim 1, having substantially the same putative structure and/or structural motif.

**3.** The nucleic acid molecule according to claim 1 or 2, wherein a sequence of the nucleic acid molecule comprises a sequence substantially having a homology to a sequence represented by SEQ ID NO: 6.

**3.** The nucleic acid molecule according to claim 1 or 2, wherein a sequence of the nucleic acid molecule comprises a sequence having a homology of at least 80% to a sequence represented by SEQ ID NO: 6.

**4.** The nucleic acid molecule according to claim 1 or 2, composed of a sequence obtained by deletion, substitution, and/or addition of one or more bases in a sequence represented by SEQ ID NO: 6.

**5.** The nucleic acid molecule according to any one of claims 2 to 4, comprising a sequence "GCGAGAA" as the structural motif.

**6.** The nucleic acid molecule according to claim 1, wherein a sequence of the nucleic acid molecule comprises a sequence represented by SEQ ID NO: 6.

**7.** The nucleic acid molecule according to claim 1, wherein a sequence of the nucleic acid molecule is composed of a sequence represented by SEQ ID NO: 6.

**8.** The nucleic acid molecule according to claim 1 or 2, wherein a sequence of the nucleic acid molecule comprises a sequence substantially having a homology to a sequence represented by SEQ ID NO: 7.

**9.** The nucleic acid molecule according to claim 1 or 2, wherein a sequence of the nucleic acid molecule comprises a sequence having a homology of at least 80% to a sequence represented by SEQ ID NO: 7.

**10.** The nucleic acid molecule according to claim 1 or 2, composed of a sequence obtained by deletion, substitution, and/or addition of one or more bases in a sequence represented by SEQ ID NO: 7.

**11.** The nucleic acid molecule according to claim 1 or 2, wherein a sequence of the nucleic acid molecule comprises a sequence represented by SEQ ID NO: 7.

**12.** The nucleic acid molecule according to claim 1, wherein a sequence of the nucleic acid molecule is composed of a sequence represented by SEQ ID NO: 7.

**13.** Use of the nucleic acid molecule according to any one of claims 1 to 12 to detect a compound having the 2,4,6-trinitrophenyl skeleton.

**14.** The use according to claim 13, wherein the compound is 2,4,6-trinitrotoluene.

**15.** A method for detecting a compound having a 2,4,6-trinitrophenyl skeleton, wherein the nucleic acid molecule according to any one of claims 1 to 12 is used.

**16.** The method according to claim 15, wherein 2,4,6-trinitrotoluene is detected by the method.
